**Europäisches Patentamt**

**European Patent Office**

**Office européen des brevets**

(19)

(11) Publication number : **0 364 012 B1**

(12) # EUROPEAN PATENT SPECIFICATION

(45) Date of publication of patent specification :
**07.01.93 Bulletin 93/01**

(51) Int. Cl.[5] : **C07C 303/24,** C07C 305/06,
C07C 309/31, C07C 43/11,
C07C 43/20, C11D 1/00

(21) Application number : **89202068.6**

(22) Date of filing : **09.08.89**

(54) **A process for the preparation of surfactants having improved physical properties.**

(30) Priority : **09.08.88 US 230808**

(43) Date of publication of application :
**18.04.90 Bulletin 90/16**

(45) Publication of the grant of the patent :
**07.01.93 Bulletin 93/01**

(84) Designated Contracting States :
**AT BE CH DE ES FR GB GR IT LI LU NL SE**

(56) References cited :
**EP-A- 0 299 370**
**AT-B- 353 761**
**DE-A- 3 431 156**
**GB-A- 2 194 536**

(56) References cited :
**US-A- 3 636 034**
**US-A- 3 839 318**
**US-A- 4 317 938**
**US-A- 4 424 169**
**US-A- 4 474 678**
**US-A- 4 721 816**

(73) Proprietor : **SHELL INTERNATIONALE**
**RESEARCH MAATSCHAPPIJ B.V.**
**Carel van Bylandtlaan 30**
**NL-2596 HR Den Haag (NL)**

(72) Inventor : **Lutz, Eugene Frederick**
**11135 Tupperlake**
**Houston Texas 77042 (US)**
Inventor : **Kravetz, Louis**
**9714 Checkerboard**
**Houston Texas 77096 (US)**

## Description

This invention relates to certain surfactant compounds and to a process for their preparation.

Surface active agents (or surfactants) are compounds which combine in one organic molecule at least one water soluble or "hydrophilic" moiety (or simply a "hydrophile") with at least one water-insoluble or "hydrophobic" moiety (a "hydrophobe"). The present invention specifically relates to surfactants having an organic hydrophobe of particularly desirable carbon chain structure.

The carbon chain structure of the surfactant hydrophobe contributes to both the chemical and physical properties of the surfactant molecule. For instance, it is known that surfactants comprising linear (straight-chain) hydrocarbyl hydrophobes have very desirable biodegradation properties. These surfactants are relatively rapidly and completely degraded under conditions encountered in common biotreaters. Biodegradability of surfactant molecules, particularly the hydrophobic moieties of surfactant molecules, in aqueous effluents is of course a matter of environmental concern. From the standpoint of their biodegradability, surfactants having linear carbon chain hydrophobes are recognized in the art as having a clear commercial advantage over their conventional branched carbon chain counterparts.

It is also known, however, that the surfactants with linear hydrocarbyl hydrophobes often do not exhibit the physical properties which are most desirable for purposes of their formulation and use. In the case of liquid detergent products such as liquid dishwashing products and liquid laundry detergent products, the linear hydrophobe surfactants have less liquidity (are more subject to gelling or solidification) and are less soluble and/or dispersible in typical liquid formulations than would be desirable. In the case of solid products such as laundry powders, the conventional linear hydrophobe surfactants frequently lack the desired degree of dispersibility in aqueous wash solutions.

The principle object of this invention is a relatively biodegradable surfactant which possesses the physical properties most desirable for formulation of detergent products. A product which combines the biodegradability of conventional linear hydrophobe surfactant molecules with the formulatability characteristics of conventional branched hydrophobe surfactant molecules would be highly desirable.

The present invention provides a class of biodegradable surfactants with improved formulatability and physical properties.

In one respect, the invention is an improved process for the preparation of surfactants from predominantly linear olefins. In the present specification with the term "predominantly" is meant at least 50%. In conventional surfactant preparation processes of interest to the invention, a higher carbon number olefin (particularly a predominantly linear higher olefin) is reacted with one or more hydrophilic moieties or precursors thereof to form the hydrophobe-hydrophile combination responsible for the product's surface active properties. The improvement associated with the present invention particularly comprises an ethylation reaction step in which the predominantly linear higher olefin feedstock is first reacted with ethylene to yield a mixture of lightly-branched mono-olefins, preferably in the carbon number range from 8 to 24, and preferably having an average carbon number between 0.2 and 4 greater than that of the olefin feedstock. The resulting ethylated olefin then serves as starting material for conversion to surfactant, for instance, in one of the conventional surfactant preparation processes. The ethylation reaction step results in a mixture of lightly-branched olefin molecules from which are derived surfactants having improved physical properties and formulatability. Surprisingly, it is found that the nature and the extent of branching in the ethylated olefin molecules do not significantly diminish the biodegradability of surfactant derivatives of the ethylated olefins, relative to derivatives of more linear olefins of corresponding carbon number.

Therefore the invention relates to a process for the preparation of a biodegradable surfactant, which comprises: (a) reacting an olefinic starting material comprising one or more predominantly linear olefins, with 6 to 22 carbon atoms per molecule, less than 10% of the molecules having more than one branch in the carbon chain, in the presence of an olefin ethylation catalyst, with sufficient ethylene to convert at least 5% of the olefinic starting material into a mixture of ethylated olefins having an average of between 1 and 2 mols of added ethylene per mol of ethylated olefins, (b) converting ethylated olefins produced in step (a) into surfactant.

The conditions placed upon the "higher" carbon number olefin reactant for the ethylation reaction step are of critical importance to the invention. Suitable olefin reactants for the ethylation reaction step of the invention necessarily comprise one or more $C_6$ to $C_{22}$ mono-olefins. From the standpoint of the demand for product surfactants, preference exists for an olefin reactant comprising one or more olefins in the carbon number range from 8 to 18, while olefins in the carbon number range from 10 to 18 are considered most preferred. In another respect, the benefits of the invention are best realized by its application to olefins in the upper carbon number levels of the indicated ranges, for instance olefins having from 12 to 22 carbon atoms. As a rule, the higher the carbon number of the surfactant derivative, the more important are improvements in physical properties and formulatability. Still many of the improved properties, for example improved solubility, are important

2

throughout the broader carbon number range.

From the standpoint of process efficiency, particularly in distillation or other separation steps which may be carried out following the ethylation reaction and also in connection with subsequent surfactant conversion steps, the olefin reactant preferably consists essentially of olefins in the specified suitable and preferred carbon number ranges. Most preferred is an olefin reactant which consists essentially of olefins within a range of at most three carbon numbers (e.g., a $C_{10}$ to $C_{12}$ range, a $C_{12}$ to $C_{14}$ range, a $C_{13}$ to $C_{15}$ range, a $C_{14}$ to $C_{16}$ range, etc.).

Suitable $C_6$ to $C_{22}$ olefins for introduction into the ethylation reaction step of the invention are necessarily of predominantly linear (i.e., straight-chain) structure, that is, at least 50 percent, preferably at least 70% and most preferably at least 90%, of the molecules have a linear carbon chain. The $C_6$ to $C_{22}$ olefin molecules which have a branched carbon chain are at most only very lightly branched. In this regard, less than 10 percent, preferably less than 5% and most preferably less than 2%, of the olefin reactant molecules have more than one branch in the carbon chain. Still more preferred are olefin reactants wherein any branches in the molecule consist of only one or two carbon atoms. Greater degrees of branching in the olefin reactant structure result in the preparation of less biodegradable surfactants.

The olefinic double bond is suitably located at any position in the molecule; both alpha-olefins and internal olefins may be utilized.

Suitable predominantly linear olefin reactants are available commercially. Specific procedures for preparing suitable olefins from ethylene are described in U.S. Patents Nos. 3,676,523, 3,686,351, 3,737,475, 3,825,615, and 4,020,121.

Also critical to the invention is the conversion of the specified $C_6$ to $C_{22}$ olefins in an ethylation reaction step, wherein the higher olefin reactant is contacted and reacted with ethylene. This ethylation reaction step introduces the desired type and degree of branching into the higher olefin carbon chain.

For olefin ethylation, the higher olefin reactant is contacted and reacted with ethylene in the presence of an effective amount of an ethylation reaction catalyst. Ethylation (alkylation) catalysts suitable for such a reaction are known in the art, and the selection of the particular catalyst is not considered critical to this invention. A preferred class of ethylation catalysts comprises one or more of the alkali metals (i.e., one or more of lithium, sodium, potassium, rubidium, and cesium). For convenience and efficiency, the one or more alkali metals are preferably supported on a solid catalyst support, e.g., carbon, alumina, silica, aluminosilicates, alkali metal carbonates, talc, and the like. Very good results have been obtained with alkali metal, e.g., potassium, supported on alumina. Such catalysts commonly contain 1 to 20 percent by weight (%w) alkali metal(s), preferably 5 to 15%w alkali metal(s).

As a general rule, the ethylene and higher olefin are suitably contacted substantially in the liquid phase at a temperature in the range from 40 to 200 °C, preferably a temperature in the range from 50 to 150 °C, and most preferably a temperature in the range from 60 to 120 °C. Pressure is preferably maintained above 3.5 bar, with pressures in the range from 7 to 35 bar considered more preferred and pressures in the range from 10 to 14 bar considered most preferred. The ethylation reaction is preferably continued until at least 5%, most preferably between 10 and 30% of the olefin reactant has undergone ethylation. Greater levels of reactant conversion are suitable, as long as the requirement is satisfied for an ethylated product wherein the average number of added ethylene groups per ethylated olefin molecule is between 1 and 2.

The ethylation reaction is suitably carried out in either a batch or continuous mode. However, in one particularly desirable embodiment, the higher olefin is ethylated in a continuous process with only a low conversion in each pass through an ethylation reactor containing the catalyst. The low conversion reaction minimizes the production of ethylated olefin characterized by addition of multiple ethylene molecules to a single olefin molecule, and thus minimizes the production of ethylated olefin molecules having more than one branch in the carbon chain and/or having branches with more than 2 carbon atoms.

The relative amounts of higher olefin reactant and ethylene are also necessarily limited in order to produce an ethylation product which has the desired degree of branching in the carbon chain. In a broad sense, the higher olefin is contacted with sufficient ethylene for sufficient time to yield a mixture of ethylated olefins characterized as the addition product of an average of between 1 and 2 mols of ethylene per mol of higher olefin. Addition of greater amounts of ethylene to the olefin decrease the biodegradability of the surfactant to which the olefin in converted.

An excess of olefin reactant may be used in the ethylation reaction in order to enhance the selectivity of the ethylation reaction for the production of ethylated products having only one added ethyl branch in the molecule. Preferably, the higher olefin reactant is used in such an excess that of the higher olefin reactant molecules that undergo reaction with ethylene, no more than 50% react with two or more ethylene molecules to yield ethylated olefins having more than one added ethyl branch and/or an added branch of more than 2 carbon atoms. More preferably, higher olefin reactant is applied in such an excess that no more than 25%, and most preferably no more than 15% of the ethylated olefins have more than one added ethyl branch and/or added branch of more than 2 carbon atoms. Typically, these preferences with regard to ethylated olefins wherein no more than 50%, 25% or 15% of the ethylated olefin molecules have multiple added ethylene branches and/or branches of more than 2 carbon atoms each, correspond to use in the ethylation reaction of higher olefin reactant and ethylene in a molar ratio which is at least 1:1, 3:1 or 6:1, respectively. Maintaining a relatively low conversion, e.g., 10-30%, of olefin reactant during ethylation also aids in preventing the formation of polyethylated products.

Additionally, or alternatively, the ethylation reaction product may be subjected to distillation or other separation means to separate the mono-ethylated molecules from polyethylated molecules of the product mixture. Moreover, unreacted higher olefin may be separated, by distillation or the like, from the ethylated olefin product before conversion to surfactant. Excess unreacted olefin may, however, suitably be left in the product, in which case the product will be a blend, containing molecules having a hydrophobe derived from the unreacted higher olefin with molecules having a hydrophobe derived from the ethylated olefins. Such blends are regarded as coming within this invention.

A particularly preferred ethylated olefin for conversion to surfactant according to the process of this invention is one which has been distilled to separate both unreacted olefin reactant and poly-ethylated products to sufficient extent that the ethylated product is accompanied by less than 15%w, particularly less than 10%w, of unreacted (unethylated) olefin reactant and also by less than 15%, particularly less than 10%w, of polyethylated molecules.

The olefin ethylation step accomplishes both the isomerization of the double bond in the higher olefin molecules and the addition of one or more molecules of ethylene to a fraction of the higher olefin molecules. As a rule, the resulting ethylated olefin molecule is typically characterized by an internal double bond and by one or more added branches of two carbon atoms each. To a substantially lesser degree, the product may also contain molecules having a terminal double bond and/or added branch(es) with more than two carbon atoms each.

Olefin mixtures resulting from the ethylation step are subsequently converted to any of a broad range of surfactants, including nonionic, anionic, cationic, and amphoteric surfactants.

In general terms, the ethylated olefin mixture serves as a surfactant intermediate. More specifically, the ethylated olefin mixture serves as the basis for the hydrophobic moiety of the surfactant molecule, with the addition during the said conversion of one or more hydrophilic moieties. Neither the particular surfactant to which the ethylated olefin is converted, nor the means of such conversion, is considered critical to the present invention, provided that it does not rearrange the skeletal structure of the molecule to the extent that the surfactant product is characterized by an average of more than 2 branches in the carbon chain having a total of more than 4 carbon atoms.

In certain preferred embodiments, the ethylated olefins are converted to alkanols, which are in turn converted to nonionic surfactants, for instance alkanol alkoxylates, alkyl polyglucosides, etc.

Conversion of higher olefins to alkanols is conveniently accomplished, for example, by hydroformylation, by oxidation and hydrolysis, by sulphation and hydration, by epoxidation and hydrogenation, or the like. Particularly beneficial highly linear alkanol products are prepared by hydroformylation of the olefins upon reaction with hydrogen and carbon monoxide according to the Oxo process. Particularly useful for purposes of the invention is the "modified Oxo process", using a phosphine, phosphite, arsine or pyridine ligand modified cobalt catalyst, as described in U.S. Patents Nos. 3,231,621, 3,239,566, 3,239,569, 3,239,570, 3,239,571, 3,420,898, 3,440,291, 3,448,158, 3,448,157, 3,496,203, and 3,496,204, 3,501,515, and 3,527,818, the disclosures of which are incorporated herein by this reference.

Conversion of the alkanols to nonionic alkoxylate surfactants is accomplished by reaction with an alkylene oxide such as ethylene oxide, propylene oxide, and their mixtures. Processes for the alkoxylation of higher alkanols are well known in the art and are exemplified by the processes described in U.S. Patent Nos. 4,721,816 and 4,474,678, the disclosures of which are incorporated herein. Preparation of the nonionic alkyl polysaccharide surfactants is accomplished by reaction of alkanols with glucose or the like according to the teachings of U.S. Patents Nos. 2,974,134, 3,640,998, 3,839,318, 3,314,936, 3,346,558, 4,011,389, and 4,223,129, incorporated herein by reference. Other nonionics which can be prepared via the process of the invention are exemplified by alkyl amines (derived, for instance, from reaction of an alkanol with ammonia or a lower (e.g., $C_1$ to $C_4$) alkyl amine), alkyl amine oxides (such as result from conversion of alkyl amines with hydrogen peroxide), alkyl amine alkoxylates (as are prepared by reaction of an alkyl amine with an alkylene oxide such as ethylene oxide, propylene oxide, or their mixtures), and carboxylic acid alkoxylates (derived via alkoxylation of corresponding carboxylic acids).

Surfactant conversion may further comprise the conversion of the alkanols or the nonionic alkanol ethoxylates to anionic surfactant derivates, including, for instance, the alkanol ethoxysulphates (prepared from corresponding alkanol ethoxylates by sulphation with $SO_3$), the alkanol ethoxycarboxylates (derived from the al-

EP 0 364 012 B1

kanol ethoxylates by reaction with chloroacetic acid), the alkanol ethoxysulphonates (prepared by sodium sulphite addition to alkanol ethoxysulphates), and the alkyl (or alkyl ethoxy) glycerol ether sulphonates (prepared as described in U.S. Patents Nos. 3,024,273 and 3,228,979, the teachings of which are incorporated herein).

In other preferred embodiments, the ethylated olefins are directly converted to anionic surfactants, for instance, to olefin sulphonates (by reaction of the olefin with sulphur trioxide). Still other anionic surfactants suitably prepared under this invention are the alkanol sulphates (by reaction of the alkanol with a sulphating agent such as sulphur trioxide, chlorosulphonic acid or sulphamic acid), the paraffin sulphonates (prepared by hydrogenation of the ethylated olefin to paraffin, followed by reaction with sulphur dioxide and oxygen under UV light), and the carboxylic acids and their derivatives such as alkyl methyl ester sulphonates (prepared by sulphur trioxide sulphonation of a methyl ester of a carboxylic acid derived from the ethylated olefin). Paraffin or Koch acids are carboxylic acids prepared by carbonylation of the ethylated olefins upon reaction with carbon monoxide and water in the presence of an acid catalyst.

Ethylated olefins are also very suitably utilized for alkylation of aromatics such as (optionally alkyl-substituted) phenols, benzene, toluene, and the xylenes, which are then converted to alkylaryl sulphonates, alkylaryl alkoxylates (e.g., alkyl phenol ethoxylates), and other surfactants by well-know procedures.

Conversions of ethylated olefins to cationic surfactants coming within the invention are illustrated by preparation of dialkyl sulphonium salts (upon the conversion of ethylated olefins to dialkyl sulphides, followed by reaction with alkyl halides), and by the synthesis of quaternary alkyl amines (such as result from the reaction of dimethyl alkyl amines and dialkyl monomethyl amines, derived from ethylated olefins).

Amphoteric surfactants suitably prepared from ethylated olefins according to this invention include aminoacids such as the alkyl betaines and the alkyl sulphobetaines.

A process wherein the conversion of ethylated olefins to surfactants comprises steps for both the conversion of the ethylated olefins to alkanols and for the conversion of the resulting alkanols to amines by reaction with ammonia or lower (e.g., $C_1$ to $C_4$) alkyl amine(s), is very useful for the preparation of surfactants in each of the nonionic, anionic, cationic and amphoteric classes.

The number of branches in the hydrophobe structure, the number of carbon atoms found in these branches, and the positioning of the branches along the carbon chain are all believed to contribute to the desirable physical properties and formulatability of the surfactant products of the invention. The typical highly branched hydrophobe surfactants of the prior art are derived from oligomers (dimers, trimers, tetramers, etc.) of propylene, butylenes, etc., and typically have an average of more than 2, typically between 2 and 4, branches in the hydrophobe structure. Relative to these prior art olefin intermediates and surfactants, the ethylated olefins intermediates and the surfactant products of the invention are "lightly branched" with both a lesser number of branches and a lesser number of total carbon atoms found in branches off the principal carbon chain.

Surfactants prepared according to the invention are characterized by a high level of biodegradability, and also by improved physical properties which are reflected in benefits in handling, formulation and use. Relative to the more linear hydrophobe biodegradable surfactants of the prior art, the products of the invention have enhanced liquidity (i.e., remain pourable liquids at lower temperatures), enhanced solubility in aqueous media at a given temperature, and/or increased rates of solubility and dispersion into aqueous media.

The products of the invention generally serve the same function as their conventional counterparts when applied either alone or in combinations (formulations) with other surfactants and/or with other known components of surfactant and detergent formulations. They may, for instance, suitably be substituted, in place of such counterparts, into conventional formulations and end uses. In this respect, an alcohol ethoxylate prepared according to the invention from a lightly branched ethylated olefin intermediate can be substituted into a formulation in place of a conventional alcohol ethoxylate component derived from a more highly linear or more highly branched conventional olefin. Alternatively, products of this invention may be mixed with their conventional counterparts to provide a mixture having improved physical properties. For instance, lightly-branched hydrophobe alkanol ethoxylates of the invention may be blended with linear hydrophobe alkanol ethoxylates of the prior art in varying proportions to provide desired physical properties. Similarly, other nonionic, anionic and cationic surfactants prepared according to the invention can be formulated in combinations with each other and/or with prior art surfactants.

As a rule, surfactants prepared according to this invention have enhanced compatibility with the other components of multiple component detergent formulations, relative to conventional counterparts having more linear hydrophobe carbon structure.

Not unexpectedly, in some cases a surfactant prepared according to the invention imparts improved performance, for instance, from the standpoint of detergency, foam generation and stability, interfacial tension or critical micelle concentration. As an example, the low temperature detergency of alcohol sulphate surfactants of this invention is greater than that of corresponding alcohol sulphates prepared from more highly linear alcohols. Similarly, certain surfactants of the invention (particularly alcohol sulphates in formulations also con-

EP 0 364 012 B1

taining conventional alkylbenzene sulphonates) have been shown to be responsible for enhanced foam generation in aqueous solution. Still further, the surfactants of the invention have in some cases been found to have less sensitivity to water hardness, i.e., their detergency is less influenced by the presence of calcium and magnesium ions, relative to conventionally derived surfactants. However, in many other cases, the performance of surfactants of the invention in typical end uses and formulations is no better than that of corresponding surfactants of the prior art. Further investigation of the performance of surfactants of the invention and their formulations, as well as optimization of the surfactants for a given service, will be well within the ability of those of ordinary skill in the art.

It should be noted, however, with respect to the preparation and formulation of products which are more economical and/or have improved performance, that such improvements can be realized in a manner which depends only on surfactant physical properties and formulatability and is essentially independent of any improvement in surfactant performance. It is often the case in formulating economical, efficient, biodegradable detergent products that the components and combinations which give an optimum performance or a most cost-effective performance for a particular service do not satisfy practical physical property constraints. For instance, the optimum performance formulation for a given application may not satisfy a requirement for a product that is a stable, flowable, one-phase liquid solution or dispersion over a range of temperatures. By providing surfactants of improved formulatability, the invention expands the opportunities for development of formulations which satisfy independent criteria placed on detergent performance, biodegradability, physical properties and production costs. As a specific example, it is now possible by applying the invention to prepare biodegradable formulations wherein one or more of the surfactant components have a greater hydrophobe carbon number or are present in greater quantity than was possible with corresponding prior art surfactants which were prepared from linear olefins and which have more limited solubility and liquidity. Such surfactants of higher carbon number have become of increasing interest in recent years for use in surfactant assisted enhanced oil recovery applications.

The invention is further described with reference to the following examples, which are intended to more particularly illustrate certain preferred embodiments of the invention without limiting its broader scope.

Examples 1-3 -- Ethylation of Olefins

These examples illustrate a series of ethylation reactions in which ethylene was contacted and reacted with a substantially linear higher carbon number olefin reactant.

In Example 1, the olefin reactant was a mixture of $C_{11}$ and $C_{12}$ (about 50% $C_{11}$ and 50% $C_{12}$) internal olefins. At least 95% of the olefin molecules had linear carbon chains. 442 grams of the olefin was charged to a one-liter autoclave, under nitrogen blanket, together with 18 grams of a supported mixed alkali metal catalyst (predominantly potassium metal supported on an alumina extrudate). For reasons of safety, the catalyst was added to the nitrogen blanketed autoclave prior to the addition of the olefin. After thoroughly flushing with nitrogen and evacuating, the autoclave was charged 35 bar ethylene. The autoclave contents were then heated under stirring to about 90 °C. As the ethylation reaction commenced, ethylene was added to maintain a total pressure in the range from 35-42 bar. As the reaction rate slowed, pressure was increased to about 56-70 bar. After about 4.5 hours, the reaction was terminated and the autoclave contents allowed to cool to room temperature. A total of 495 grams of reactants and catalyst were removed and then filtered to separate catalyst. The liquid product was distilled to separate out both unreacted olefin reactant and polyethylated products. A distillation cut was made to obtain 95.7 grams of a mixture consisting essentially of $C_{13}$ and $C_{14}$ internal olefins.

For Example 2, the ethylation of a reactant consisting essentially of 1-tetradecene was carried out according to the same procedures, and using the same quantities of catalyst and olefin reactant, as described for Example 1. At least 96% of the higher olefin reactant molecules had linear carbon chains. Following filtration for catalyst removal, the liquid reaction mixture was distilled under vacuum to recover 97.4 grams of a product cut consisting essentially of $C_{16}$ internal olefins.

Example 3 illustrates a continuous ethylation process step applied to a reactant consisting essentially of 1-octene (at least about 96% of the molecules of which had linear carbon chains). The olefin reactant 1-octene was presaturated with ethylene at 14 bar and 22 °C, and this reactant mixture was then passed in sequence through an alumina bed (to remove feed impurities such as oxygenates), and through a fixed bed reactor of the same alumina-supported alkali metal catalyst used in Example 1, at a liquid hourly space velocity of 2.0. Samples of product exiting the reactor bed were taken at intervals over a 150 hour run. Process conditions and results for the continuous ethylation step are presented in the following table.

| Run Hours | Temp. °C | Pressure bar | Conversion | | Selectivities | | |
|---|---|---|---|---|---|---|---|
| | | | 1-Octene | Ethylene | $C_{10}$ | $C_{12}$ | $C_{14}^+$ |
| 3 | 52 | 35 | 21.7 | 97.5 | 92.1 | 7.7 | 0.3 |
| 23 | 50 | 35 | 18.5 | 88 | 89.8 | 9.4 | 0.9 |
| 24 | 59 | 35 | | | | | |
| 27 | 59 | 35 | 20.3 | 92.3 | 89.2 | 9.9 | 0.9 |
| 46 | 59 | 35 | 20.3 | 89 | 89.1 | 6.7 | 4.2 |
| 53 | 63 | 35 | | | | | |
| 70 | 63 | 35 | 17.9 | 78 | 82.0 | 14.3 | 3.7 |
| 71 | 63 | 53 | | | | | |
| 74 | 63 | 53 | 15.5 | 72 | 82.6 | 13.9 | 3.5 |
| 75 | 72 | 53 | | | | | |
| 77 | 72 | 53 | 18.5 | 82 | 86.7 | 9.7 | 3.6 |
| 78 | 86 | 35 | | | | | |
| 84 | 86 | 35 | 24.0 | 94 | 88.5 | 8.8 | 2.7 |
| 102 | 86 | 35 | 26.3 | 98 | 84.3 | 13.5 | 2.2 |
| 126 | 86 | 35 | 23.5 | 98 | 84.7 | 12.2 | 3.0 |
| 131 | 89 | 35 | | | | | |
| 150 | 89 | 35 | 24.9 | 97 | 84.5 | 13.1 | 2.3 |

Examples 4 and 5 -- Conversion of ethylated olefins to alcohols; positions of branching in the alcohol molecule

In Example 4, the mixture of $C_{13}$ and $C_{14}$ internal olefins prepared by ethylation as described in Example 1 was converted to a mixture of $C_{14}$ and $C_{15}$ alkanols by hydroformylation using the modified Oxo process. A total of 93.5 grams of the internal olefins was reacted with a mixture of hydrogen and carbon monoxide (in 2:1 molar ratio) in the presence of a phosphine modified cobalt catalyst at a temperature of 205 °C and a pressure of 77 bar for 6 hours. On completion of the reaction, the crude product (108 grams) was combined with that of an identical batch hydroformylation and the combination was treated by vacuum distillation, including wiped-film evaporation, to recover 133 grams of a high purity (98.3%) mixture of $C_{14}$ and $C_{15}$ alkanols, in approximately equimolar amounts.

The lightly-branched alkanol mixture produced in this Example 4 was a clear liquid at zero degrees centigrade. In comparison, a $C_{14}/C_{15}$ alkanol mixture prepared by hydroformylation of essentially linear $C_{13}$ and $C_{14}$ internal olefins has a melting point range from 15 to 36 °C.

$C_{13}$-NMR analysis of the product mixture of Example 4 showed the following distribution of positions of branching in the alkanol molecules:

| Location of Branching | mol % |
|---|---|
| 2-position | 12 |
| 3-position | 1 |
| 4-position | 49 |
| 5+-position | 38 |

In Example 5, 94.5 grams of the $C_{16}$ internal olefin prepared as described in Example 2 were hydroformylated under the procedures described in Example 4 to prepare $C_{17}$ alkanol. The crude $C_{17}$ alkanol product was distilled and filtered, leaving a clear and colorless product which was a liquid at 25 °C. NMR analysis showed the following distribution of branching in the alkanol molecules:

| Location of Branching | mol % |
|---|---|
| 2-position | 11 |
| 3-position | 1 |
| 4-position | 45 |
| 5+-position | 43 |

The frequency of branching at a position in the molecule remote from the position of the hydroxyl group confirms that the alcohols can be readily converted to derivatives, such as carboxylates, sulphates and ethoxylates.

Example 6 -- Conversion of alcohols to alcohol sulphates;

For Example 6, the mixture of $C_{14}$ and $C_{15}$ alcohols prepared according to the procedures of Example 4 was reacted with chlorosulphonic acid to form the corresponding alcohol sulphuric acids, which were then neutralized to form the alcohol sulphate sodium salts. More specifically, 100 millimols of the $C_{14}/C_{15}$ alkanol mixture were dissolved in 25 ml of dry methylene chloride in a 100 ml flask. Dry nitrogen gas was bubbled through the solution for one half hour. Then 105 millimols of chlorosulphonic acid was dripped into the alcohol solution with vigorous stirring at a rate sufficient to maintain the reaction temperature at 25-30 °C. Dry nitrogen was bubbled through the stirred reaction mixture for an additional 30 minutes to purge hydrogen chloride which had formed as a reaction byproduct. The reaction mixture was then added dropwise to a solution of 105 millimols of sodium hydroxide dissolved in sufficient deionized water and the methylene chloride evaporated under reduced pressure. This produced a solution containing about 20-25% by weight of the alcohol sulphate sodium salt.

Example 7 -- Biodegradability of alcohol sulphates

The biodegradability of several different alcohol sulphate surfactants was tested under the standard biochemical oxygen demand (BOD) test procedure described in Standard Methods for the Examination of Water and Wastewater, 15th Edition, prepared and published jointly by the American Public Health Association, American Water Works Association, and Water Pollution Control Federation. Results of the tests are reported in terms of measured biochemical oxygen demand over 30 days divided by chemical oxygen demand (for full degradation), multiplied by 100.

For the alcohol sulphate surfactants of the invention, prepared according to Example 6, biodegradability in the BOD test was 81%.

For comparative purposes, the standard BOD test was also carried out to determine the biodegradability the alcohol sulphate product prepared by sulphation of a commercial mixture of $C_{14}$ and $C_{15}$ alcohols, which had been derived from highly linear olefins via the modified Oxo process. Biodegradability was measured at 84%. These comparative tests establish that the biodegradability of the surfactants of the invention is not meaningfully different from the biodegradability of surfactants of the prior art derived in a similar fashion but from highly linear olefins.

Also for comparison, the standard BOD test was applied to highly branched, $C_{13}$ hydrophobe alcohol sulphate surfactants derived from a commercial propylene tetramer by hydroformylation and sulphation. Under the same test conditions, biodegradability of the highly branched carbon chain alcohol sulphate surfactants was only 41%. This comparison establishes that the biodegradability of the surfactants of the invention is substantially superior to that of the sulphates of highly branched alcohols of the prior art.

Example 8 -- Dispersion rates for alcohol sulphates

A comparison was made of dispersion rates for lightly branched alcohol sulphates prepared according to the invention and for conventional alcohol sulphates prepared from highly linear alcohols. A mixture of $C_{14}$ and $C_{15}$ alcohol prepared according to the procedures of Example 4 was converted to alcohol sulphates surfactants according to this invention. For sulphation, the procedures of Example 6 were followed with an additional step in which the chlorosulphonic acid reaction product was dissolved in sufficient methylene chloride to give a concentration of 0.37 grams of alcohol sulphate per ml of methylene chloride. Then 50.5 ml of this solution was neutralized upon addition to a slurry of 80 grams of a mixture of sodium carbonate and sodium bicarbonate (in a 1:2 weight ratio) in 300 ml of dry methylene chloride. The neutralization product was an alcohol sulphate sodium salt. Methylene chloride was then evaporated from the slurry, leaving a carbonate residue containing approximately 20%w of adsorbed alcohol sulphate.

Rate of dispersion of the alcohol sulphates of this invention was determined by adding 0.1 grams of the carbonate/alcohol sulphate material to 100 ml of deionized water and measuring light transmittance through the mixture at temperatures in the range from 10 to 30 °C. After several minutes, light transmission through the mixture was greater than 90%. For comparison, carbonate/alcohol sulphate materials prepared in this manner from conventional highly linear alcohols were similarly tested and found to give a light transmission value of only about 20%. This comparison shows that the surfactant derived according to this invention is substantially more dispersible and soluble in water than the corresponding surfactant prepared from highly linear alcohols.

Example 9 -- Conversion of alcohols to alcohol ethoxylates and alcohol ethoxysulphates

This example illustrates the conversion of a mixture of $C_{14}$ and $C_{15}$ alcohols prepared according to the procedures of Example 4 to two nonionic alcohol ethoxylate surfactants. The alcohol was reacted with ethylene oxide in the presence of a potassium hydroxide catalyst. A mixture of 102 grams of the $C_{14}/C_{15}$ alcohol mixture and 0.29 grams of 85% potassium hydroxide was heated under nitrogen sparge at 120 °C for two hours and then cooled. 100.9 grams of this mixture was added to a 300 ml autoclave reactor under nitrogen atmosphere (2.8 bar) and heated to about 155 °C. Ethylene oxide was introduced to bring pressure to about 5.6 bar and continually added during the reaction to maintain that pressure. A total of 43.9 grams of ethylene oxide was added over a period of 67 minutes. Then ethylene oxide addition was discontinued and the reactor contents were allowed to soak at temperature for an additional 30 minutes to consume unreacted ethylene oxide. At this time 95.3 grams of alcohol ethoxylate was withdrawn from the reactor. This surfactant product was analyzed to have an average of 2.4 mols of added ethylene oxide per mol of alcohol ethoxylate. Ethylene oxide addition to the reactor was again commenced and a further 32.2 grams of ethylene oxide were added over 84 minutes at 155 °C, followed by a soak time of 40 minutes. A product of the continued ethoxylation, totalling 79.3 grams, was withdrawn and analyzed to have an average of 7.9 mols of added ethylene oxide per mol of alcohol ethoxylate.

The alcohol ethoxylate surfactant product having an average of 2.4 mols of added ethylene oxide per mol of ethoxylate was sulphated for conversion to alcohol ethoxysulphate surfactant. For this purpose, 100 millimols of the alcohol ethoxysulphate was sparged with dry nitrogen gas for one half hour. Under nitrogen atmosphere, 105 millimols of chlorosulphonic acid was dripped into the ethoxy ate with vigorous stirring at a rate sufficient to maintain the reaction temperature at 25 to 30 °C. Dry nitrogen was then bubbled through the stirred reaction mixture for an additional 30 minutes. The reaction mixture was then added dropwise to a solution of 105 millimols of sodium hydroxide dissolved in deionized water to give a 20 to 25 percent active matter mixture of the alcohol ethoxysulphate.

The alcohol ethoxysulphate surfactant prepared according to this example of the invention was less viscous and had less of a tendency to form a gel than corresponding conventional alcohol ethoxysulphates derived from highly linear alcohols.

**Claims**

**Claims for the following Contracting States : AT, BE, CH, DE, FR, GB, GR, IT, LI, LU, NL, SE**

1.  A process for the preparation of a biodegradable surfactant which comprises:

    (a) reacting an olefinic starting material comprising one or more predominantly linear olefins, with 6 to 22 carbon atoms per molecule, less than 10% of the molecules having more than one branch in the carbon chain, in the presence of an olefin ethylation catalyst, with sufficient ethylene to convert at least 5% of the olefinic starting material into a mixture of ethylated olefins having an average of between 1 and 2 mols of added ethylene per mol of ethylated olefins,

    (b) converting ethylated olefins produced in step (a) into surfactant.

2.  A process according to claim 1, characterized in that from the reaction mixture of step (a) an enriched ethylated olefin product is separated containing less than 10% by weight of unreacted olefin reactant and less than 10% by weight of polyethylated olefins, and that the enriched mixture thus obtained is converted into surfactant.

3. A process according to claim 1, characterized in that in step (a) a starting material comprising predominantly linear $C_8$ to $C_{20}$ olefins, less than 10% of which having more than one branch in the carbon chain, is reacted with sufficient ethylene to convert between 10 and 40% by mol of the starting material to a mixture of ethylated olefins having an average of between 1 and 1.5 mols of added ethylene per mol of ethylated olefins; that ethylated olefins produced in step (a) are hydroformylated under formation of alkanols; and that alkanols thus formed are converted into surfactant.

4. A process according to claim 3, characterized in that from the reaction mixture obtained in step (a) an enriched ethylated olefin product is separated containing less than 10% by weight of unreacted olefin reactant and less than 10% by weight of polyethylated olefins; that the enriched mixture thus obtained is hydroformylated under formation of alkanols; and that alkanols thus formed are converted into surfactant.

5. A process according to claim 1 or 2, characterized in that ethylated olefins are converted to olefin sulphonate surfactant by reaction with sulphur trioxide.

6. A process according to claim 1 or 2, characterized in that the conversion of ethylated olefins to surfactant comprises an aromatic alkylation step for reacting the ethylated olefins with one or more aromatics selected from the groups consisting of phenols, benzene, toluene and the xylenes.

7. A process according to claim 6, characterized in that the conversion of ethylated olefins to surfactant further comprises a sulphonation step for converting the alkylated aromatics to corresponding sulphonates by reaction with a sulphonating agent.

8. A process according to claim 6, characterized in that the aromatic reactant is selected from the group consisting of phenols, and the conversion of ethylated olefins to surfactant further comprises an ethoxylation step for converting the alkylated phenols to corresponding ethoxylates by reaction with ethylene oxide.

9. A process according to claim 3 or 4, characterized in that the alkanols are converted to alkanol sulphate surfactant by reaction with a sulphation agent selected from the group consisting of sulphur trioxide, chlorosulphonic acid, sulphamic acid, and mixtures thereof.

10. A process according to claim 3 or 4, characterized in that the alkanols are converted into alkyl or alkyl ethoxy glycerol ether sulphonate surfactant.

11. A process according to claim 3 or 4, characterized in that the alkanols are converted into alkyl polysaccharide surfactant.

12. A process according to claim 3 or 4, characterized in that the conversion of alkanols into surfactants comprises a step for reacting the alkanols with one or more reactants selected from the group consisting of ammonia and $C_1$ to $C_4$ alkyl amines.

13. Surfactants obtainable by conversion of ethylated olefins produced in step (a) of the process according to claim 1 or 3.

14. Surfactants obtainable by conversion of an enriched mixture of ethylated olefins produced according to claim 2 or 4.

**Claims for the following Contracting State : ES**

1. A process for the preparation of a biodegradable surfactant which comprises:
   (a) reacting an olefinic starting material comprising one or more predominantly linear olefins, with 6 to 22 carbon atoms per molecule, less than 10% of the molecules having more than one branch in the carbon chain, in the presence of an olefin ethylation catalyst, with sufficient ethylene to convert at least 5% of the olefinic starting material into a mixture of ethylated olefins having an average of between 1 and 2 mols of added ethylene per mol of ethylated olefins,
   (b) converting ethylated olefins produced in step (a) into surfactant.

**2.** A process according to claim 1, characterized in that from the reaction mixture of step (a) an enriched ethylated olefin product is separated containing less than 10% by weight of unreacted olefin reactant and less than 10% by weight of polyethylated olefins, and that the enriched mixture thus obtained is converted into surfactant.

**3.** A process according to claim 1, characterized in that in step (a) a starting material comprising predominantly linear $C_8$ to $C_{20}$ olefins, less than 10% of which having more than one branch in the carbon chain, is reacted with sufficient ethylene to convert between 10 and 40% by mol of the starting material to a mixture of ethylated olefins having an average of between 1 and 1.5 mols of added ethylene per mol of ethylated olefins; that ethylated olefins produced in step (a) are hydroformylated under formation of alkanols; and that alkanols thus formed are converted into surfactant.

**4.** A process according to claim 3, characterized in that from the reaction mixture obtained in step (a) an enriched ethylated olefin product is separated containing less than 10% by weight of unreacted olefin reactant and less than 10% by weight of polyethylated olefins; that the enriched mixture thus obtained is hydroformylated unde? formation of alkanols; and that alkanols thus formed are converted into surfactant.

**5.** A process according to claim 1 or 2, characterized in that ethylated olefins are converted to olefin sulphonate surfactant by reaction with sulphur trioxide.

**6.** A process according to claim 1 or 2, characterized in that the conversion of ethylated olefins to surfactant comprises an aromatic alkylation step for reacting the ethylated olefins with one or more aromatics selected from the groups consisting of phenols, benzene, toluene and the xylenes.

**7.** A process according to claim 6, characterized in that the conversion of ethylated olefins to surfactant further comprises a sulphonation step for converting the alkylated aromatics to corresponding sulphonates by reaction with a sulphonating agent.

**8.** A process according to claim 6, characterized in that the aromatic reactant is selected from the group consisting of phenols, and the conversion of ethylated olefins to surfactant further comprises an ethoxylation step for converting the alkylated phenols to corresponding ethoxylates by reaction with ethylene oxide.

**9.** A process according to claim 3 or 4, characterized in that the alkanols are converted to alkanol sulphate surfactant by reaction with a sulphation agent selected from the group consisting of sulphur trioxide, chlorosuphonic acid, sulphamic acid, and mixtures thereof.

**10.** A process according to claim 3 or 4, characterized in that the alkanols are converted into alkyl or alkyl ethoxy glycerol ether sulphonate surfactant.

**11.** A process according to claim 3 or 4, characterized in that the alkanols are converted into alkyl polysaccharide surfactant.

**12.** A process according to claim 3 or 4, characterized in that the conversion of alkanols into surfactants comprises a step for reacting the alkanols with one or more reactants selected from the group consisting of ammonia and $C_1$ to $C_4$ alkyl amines.

**Patentansprüche**

**Patentansprüche für folgende Vertragsstaaten : AT, BE, CH, DE, FR, GB, GR, IT, LI, LU, NL, SE**

**1.** Verfahren zur Herstellung eines biologisch abbaubaren Surfactants, umfassend:
  (a) Umsetzen eines olefinischen Ausgangsmaterials, umfassend ein oder mehrere vorwiegend lineare Olefine mit 6 bis 22 Kohlenstoffatomen je Molekül, wobei weniger als 10 % der Moleküle mehr als eine Verzweigung in der Kohlenstoffkette aufweisen, in Anwesenheit eines Olefin-ethylierungskatalysators mit ausreichend Ethylen, um wenigstens 5 % des olefinischen Ausgansmaterials in ein Gemisch von ethylierten Olefinen mit im Mittel zwischen 1 und 2 Mol addiertem Ethylen je Mol ethylierten Olefinen umzuwandeln,
  (b) Umwandeln der in Stufe (a) hergestellten ethylierten Olefine in ein Surfactant.

**2.** Verfahren nach Anspruch 1, dadurch gekennzeichnet, daß aus dem Reaktionsgemisch von Stufe (a) ein angereichertes ethyliertes Olefinprodukt abgetrennt wird, das weniger als 10 Gew.-% an nicht-umgesetztem Olefinreagens und weniger als 10 Gew.-% an polyethylierten Olefinen enthält, und daß solcherart erhaltene angereicherte Gemisch zu einem Surfactant umgewandelt wird.

**3.** Verfahren nach Anspruch 1, dadurch gekennzeichnet, daß in Stufe (a) ein vorwiegend lineare $C_8$-$C_{20}$-Olefine umfassendes Ausgansmaterial, von welchen Olefinen weniger als 10 % mehr als eine Verzweigung in der Kohlenstoffkette aufweisen, mit ausreichend Ethylen umgesetzt wird, um zwischen 10 und 40 Mol-% des Ausgangsmaterials in ein Gemisch von ethylierten Olefinen mit im Mittel zwischen 1 und 1,5 Mol addiertem Ethylen je Mol ethylierte Olefine umzuwandeln; daß die in Stufe (a) gebildeten ethylierten Olefine unter Bildung von Alkanolen hydroformyliert werden; und daß die solcherart gebildeten Alkanole zu einem Surfactant umgewandelt werden.

**4.** Verfahren nach Anspruch 3, dadurch gekennzeichnet, daß aus dem in Stufe (a) erhaltenen Reaktionsgemisch ein angereichertes ethyliertes Olefinprodukt abgetrennt wird, das weniger als 10 Gew.-% nicht-umgesetztes Olefinreagens und weniger als 10 Gew.-% polyethylierte Olefine enthält; daß das solcherart erhaltene angereicherte Gemisch unter Bildung von Alkanolen hydroformyliert wird; und daß die solcherart gebildeten Alkanole zu einem Surfactant umgewandelt werden.

**5.** Verfahren nach Anspruch 1 oder 2, dadurch gekennzeichnet, daß die ethylierten Olefine durch Umsetzung mit Schwefeltrioxid zu einem Olefin-sulfonat-Surfactant umgewandelt werden.

**6.** Verfahren nach Anspruch 1 oder 2, dadurch gekennzeichnet, daß die Umwandlung der ethylierten Olefine zu einem Surfactant eine Aromaten-Alkylierungsstufe zur Umsetzung der ethylierten Olefine mit einem oder mit mehreren Aromaten, ausgewählt aus den aus Phenolen, Benzol, Toluol und den Xylolen bestehenden Gruppen, umfaßt.

**7.** Verfahren nach Anspruch 6, dadurch gekennzeichnet, daß die Umwandlung der ethylierten Olefine zu einem Surfactant weiterhin eine Sulfonierungsstufe zur Umwandlung der alkylierten Aromaten zu entsprechenden Sulfonaten durch Reaktion mit einem Sulfonierungsmittel umfaßt.

**8.** Verfahren nach Anspruch 6, dadurch gekennzeichnet, daß das aromatische Reagens aus der aus Phenolen bestehenden Gruppe ausgewählt wird, und daß die Umwandlung der ethylierten Olefine zu einem Surfactant weiterhin eine Ethoxylierungsstufe zur Umwandlung der alkylierten Phenole zu entsprechenden Ethoxylaten durch Umsetzung mit Ethylenoxid umfaßt.

**9.** Verfahren nach Anspruch 3 oder 4, dadurch gekennzeichnet, daß die Alkanole durch Umsetzung mit einem Sulfatierungsmittel, ausgewählt aus der aus Schwefeltrioxid, Chlorsulfonsäure, Sulfaminsäure und deren Gemischen bestehenden Gruppe, zu einem Alkanolsulfat-Surfactant umgewandelt werden.

**10.** Verfahren nach Anspruch 3 oder 4, dadurch gekennzeichnet, daß die Alkanole zu einem Alkyl- oder Alkylethoxyglycerinethersulfonat-Surfactant umgewandelt werden.

**11.** Verfahren nach Anspruch 3 oder 4, dadurch gekennzeichnet, daß die Alkanole zu einem Alkylpolysaccharid-Surfactant umgewandelt werden.

**12.** Verfahren nach Anspruch 3 oder 4, dadurch gekennzeichnet, daß die Umwandlung der Alkanole zu Surfactants eine Stufe zum Reagieren der Alkanole mit einem oder mit mehreren Reagenzien, ausgewählt aus der aus Ammoniak und $C_1$-$C_4$-Alkylaminen bestehenden Gruppe, umfaßt.

**13.** Surfactants, erhältlich durch Umwandlung von ethylierten Olefinen, hergestellt in Stufe (a) des Verfahrens nach Anspruch 1 oder 3.

**14.** Surfactants, erhältlich durch Umwandlung eine angereicherten Gemisches von ethylierten Olefinen, hergestellt nach Anspruch 2 oder 4.

**Patentansprüche für folgenden Vertragsstaat : ES**

**1.** Verfahren zur Herstellung eines biologisch abbaubaren Surfactants, umfassend:
(a) Umsetzen eines olefinischen Ausgangsmaterials, umfassend ein oder mehrere vorwiegend lineare Olefine mit 6 bis 22 Kohlenstoffatomen je Molekül, wobei weniger als 10 % der Moleküle mehr als eine Verzweigung in der Kohlenstoffkette aufweisen, in Answesenheit eines Olefin-ethylierungskatalysators mit ausreichend Ethylen, um wenigstens 5 % des olefinischen Ausgansmaterials in ein Gemisch von ethylierten Olefinen mit im Mittel zwischen 1 und 2 Mol addiertem Ethylen je Mol ethylierten Olefinen umzuwandeln,

(b) Umwandeln der in Stufe (a) hergestellten ethylierten Olefine in ein Surfactant.

2. Verfahren nach Anspruch 1, dadurch gekennzeichnet, daß aus dem Reaktionsgemisch von stufe (a) ein angereichertes ethyliertes Olefinprodukt abgetrennt wird, das weniger als 10 Gew.-% an nicht-umgesetztem Olefinreagens und weniger als 10 Gew.-% an polyethylierten Olefinen enthält, und daß solcherart erhaltene angereicherte Gemisch zu einem Surfactant umgewandelt wird.

3. Verfahren nach Anspruch 1, dadurch gekennzeichnet, daß in Stufe (a) ein vorwiegend lineare $C_8$-$C_{20}$-Olefine umfassendes Ausgansmaterial, von welchen Olefinen weniger als 10 % mehr als eine Verzweigung in der Kohlenstoffkette aufweisen, mit ausreichend Ethylen umgesetzt wird, um zwischen 10 und 40 Mol-% des Ausgangsmaterials in ein Gemisch von ethylierten Olefinen mit im Mittel zwischen 1 und 1,5 Mol addiertem Ethylen je Mol ethylierte Olefine umzuwandeln; daß die in Stufe (a) gebildeten ethylierten Olefine unter Bildung von Alkanolen hydroformyliert werden; und daß die solcherart gebildeten Alkanole zu einem Surfactant umgewandelt werden.

4. Verfahren nach Anspruch 3, dadurch gekennzeichnet, daß aus dem in Stufe (a) erhaltenen Reaktionsgemisch ein angereichertes ethyliertes Olefinprodukt abgetrennt wird, das weniger als 10 Gew.-% nicht-umgesetztes Olefinreagens und weniger als 10 Gew.-% polyethylierte Olefine enthält; daß das solcherart erhaltene angereicherte Gemisch unter Bildung von Alkanolen hydroformyliert wird; und daß die solcherart gebildeten Alkanole zu einem Surfactant umgewandelt werden.

5. Verfahren nach Anspruch 1 oder 2, dadurch gekennzeichnet, daß die ethylierten Olefine durch Umsetzung mit Schwefeltrioxid zu einem Olefin-sulfonat-Surfactant umgewandelt werden.

6. Verfahren nach Anspruch 1 oder 2, dadurch gekennzeichnet, daß die Umwandlung der ethylierten Olefine zu einem Surfactant eine Aromaten-Alkylierungsstufe zur Umsetzung der ethylierten Olefine mit einem oder mit mehreren Aromaten, ausgewählt aus den aus Phenolen, Benzol, Toluol und den Xylolen bestehenden Gruppen, umfaßt.

7. Verfahren nach Anspruch 6, dadurch gekennzeichnet, daß die Umwandlung der ethylierten Olefine zu einem Surfactant weiterhin eine Sulfonierungsstufe zur Umwandlung der alkylierten Aromaten zu entsprechenden Sulfonaten durch Reaktion mit einem Sulfonierungsmittel umfaßt.

8. Verfahren nach Anspruch 6, dadurch gekennzeichnet, daß das aromatische Reagens aus der aus Phenolen bestehenden Gruppe ausgewählt wird, und daß die Umwandlung der ethylierten Olefine zu einem Surfactant weiterhin eine Ethoxylierungsstufe zur Umwandlung der alkylierten Phenole zu entsprechenden Ethoxylaten durch Umsetzung mit Ethylenoxid umfaßt.

9. Verfahren nach Anspruch 3 oder 4, dadurch gekennzeichnet, daß die Alkanole durch Umsetzung mit einem Sulfatierungsmittel, ausgewählt aus der aus Schwefeltrioxid, Chlorsulfonsäure, Sulfaminsäure und deren Gemischen bestehenden Gruppe, zu einem Alkanolsulfat-Surfactant umgewandelt werden.

10. Verfahren nach Anspruch 3 oder 4, dadurch gekennzeichnet, daß die Alkanole zu einem Alkyl- oder Alkylethoxyglycerinethersulfonat-Surfactant umgewandelt werden.

11. Verfahren nach Anspruch 3 oder 4, dadurch gekennzeichnet, daß die Alkanole zu einem Alkylpolysaccharid-Surfactant umgewandelt werden.

12. Verfahren nach Anspruch 3 oder 4, dadurch gekennzeichnet, daß die Umwandlung der Alkanole zu Surfactants eine Stufe zum Reagieren der Alkanole mit einem oder mit mehreren Reagenzien, ausgewählt aus der aus Ammoniak und $C_1$-$C_4$-Alkylaminen bestehenden Gruppe, umfaßt.

EP 0 364 012 B1

**Revendications**

**Revendications pour les Etats contractants suivants : AT, BE, CH, DE, FR, GB, GR, IT, LI, LU, NL, SE**

1.  Un procédé pour la préparation d'un agent tensio-actif biodégradable, qui comprend les étapes selon lesquelles :

    (a) on fait réagir une matière oléfinique comprenant une ou plusieurs oléfines principalement linéaires, ayant de 6 à 22 atomes de carbone par molécule, moins de 10 % des molécules ayant plus d'une ramification dans la chaîne carbonée, en présence d'un catalyseur d'éthylation d'oléfines, avec assez d'éthylène pour transformer au moins 5 % de la matière oléfinique de départ en un mélange d'oléfines éthylées ayant une moyenne comprise entre 1 et 2 moles d'éthylène ajouté par mole d'oléfines éthylées, et

    (b) on transforme les oléfines éthylées produites dans l'étape (a) en agent tensio-actif.

2.  Un procédé selon la revendication 1, caractérisé en ce qu'à partir du mélange de réaction de l'étape (a) on sépare un produit oléfine éthylée enrichi contenant moins de 10 % en poids de matière de départ oléfinique n'ayant pas réagi et moins de 10 % en poids d'oléfines polyéthylées et que le mélange enrichi ainsi obtenu est transformé en agent tensio-actif.

3.  Un procédé selon la revendication 1, caractérisé en ce que dans l'étape (a) une matière de départ comprenant des oléfines de $C_8$ à $C_{20}$ principalement linéaires, dont moins de 10 % ont plus d'une branche dans la chaîne carbonée, est mise à réagir avec une quantité d'éthylène suffisante pour transformer de 10 à 40 moles % de la matière de départ en un mélange d'oléfines éthylées ayant une moyenne comprise entre 1 et 1,5 mole d'éthylène ajouté par mole d'oléfines éthylées ; que les oléfines éthylées produites dans l'étape (a) sont hydroformylées avec formation d'alcanols ; et que les alcanols ainsi formés sont transformés en agent tensio-actif.

4.  Un procédé selon la revendication 3, caractérisé en ce qu'à partir du mélange de réaction obtenu dans l'étape (a) on sépare un produit oléfine éthylée enrichi contenant moins de 10 % en poids de matière de départ oléfinique n'ayant pas réagi et moins de 10 % en poids d'oléfines polyéthylées ; que le mélange enrichi ainsi obtenu est hydroformylé avec formation d'alcanols ; et que les alcanols ainsi formés sont transformés en agent tensio-actif.

5.  Un procédé selon la revendication 1 ou 2, caractérisé en ce que les oléfines éthylées sont transformées en agent tensio-actif sulfonate d'oléfine par réaction avec l'anhydride sulfurique.

6.  Un procédé selon la revendication 1 ou 2, caractérisé en ce que la conversion oléfines éthylées en agent tensio-actif comprend une étape d'alcoylation par des composés aromatiques pour faire réagir les oléfines éthylées avec un ou plusieurs composés aromatiques compris dans le groupe constitué par les phénols, le benzène, le toluène et les xylènes.

7.  Un procédé selon la revendication 6, caractérisé en ce que la conversion des oléfines éthylées en agent tensio-actif comprend aussi une étape de sulfonation pour transformer les composés aromatiques alcoylés en sulfonates correspondants par réaction avec un agent de sulfonation.

8.  Un procédé selon la revendication 6, caractérisé en ce que le corps en réaction aromatique est choisi dans le groupe constitué par les phénols et que la conversion des oléfines éthylées en agent tensio-actif comprend aussi une étape d'éthoxylation pour transformer les phénols alcoylés en éthoxylates correspondants par réaction avec l'oxyde d'éthylène.

9.  Un procédé selon la revendication 3 ou 4, caractérisé en ce que les alcanols sont transformés en agent tensio-actif sulfate d'alcanol par réaction avec un agent de sulfatation choisi dans le groupe constitué par l'anhydride sulfurique, l'acide chlorosulfonique, l'acide sulfamique et leurs mélanges.

10. Un procédé selon la revendication 3 ou 4, caractérisé en ce que les alcanols sont transformés en agent tensio-actif sulfonate d'éther d'alcoyl ou d'alcoyl éthoxy glycérol.

11. Un procédé selon la revendication 3 ou 4, caractérisé en ce que les alcanols sont transformés en agent

14

EP 0 364 012 B1

tensio-actif alcoyl polysaccharide.

12. Un procédé selon la revendication 3 ou 4, caractérisé en ce que la conversion des alcanols en agents tensio-actifs comprend une étape pour faire réagir les alcanols avec un ou plusieurs réactifs choisis dans le groupe constitué par l'ammoniac et les alcoylamines de $C_1$ à $C_4$.

13. Les agents tensio-actifs pouvant être obtenus par conversion des oléfines éthylées produites dans l'étape (a) du procédé selon la revendication 1 ou 3.

14. Les agents tensio-actifs pouvant être obtenus par conversion d'un mélange enrichi d'oléfines éthylées produit selon la revendication 2 ou 4.

**Revendications pour l'Etat contractant suivant : ES**

1. Un procédé pour la préparation d'un agent tensio-actif biodégradable, qui comprend les étapes selon lesquelles :
   (a) on fait réagir une matière oléfinique comprenant une ou plusieurs oléfines principalement linéaires, ayant de 6 à 22 atomes de carbone par molécule, moins de 10 % des molécules ayant plus d'une ramification dans la chaîne carbonée, en présence d'un catalyseur d'éthylation d'oléfines, avec assez d'éthylène pour transformer au moins 5 % de la matière oléfinique de départ en un mélange d'oléfines éthylées ayant une moyenne comprise entre 1 et 2 moles d'éthylène ajouté par mole d'oléfines éthylées, et
   (b) on transforme les oléfines éthylées produites dans l'étape (a) en agent tensio-actif.

2. Un procédé selon la revendication 1, caractérisé en ce qu'à partir du mélange de réaction de l'étape (a) on sépare un produit oléfine éthylée enrichi contenant moins de 10 % en poids de matière de départ oléfinique n'ayant pas réagi et moins de 10 % en poids d'oléfines polyéthylées et que le mélange enrichi ainsi obtenu est transformé en agent tensio-actif.

3. Un procédé selon la revendication 1, caractérisé en ce que dans l'étape (a) une matière de départ comprenant des oléfines de $C_8$ à $C_{20}$ principalement linéaires, dont moins de 10 % ont plus d'une branche dans la chaîne carbonée, est mise à réagir avec une quantité d'éthylène suffisante pour transformer de 10 à 40 moles % de la matière de départ en un mélange d'oléfines éthylées ayant une moyenne comprise entre 1 et 1,5 mole d'éthylène ajouté par mole d'oléfines éthylées ; que les oléfines éthylées produites dans l'étape (a) sont hydroformylées avec formation d'alcanols ; et que les alcanols ainsi formés sont transformés en agent tensio-actif.

4. Un procédé selon la revendication 1, caractérisé en ce qu'à partir du mélange de réaction obtenu dans l'étape (a) on sépare un produit oléfine éthylée enrichi contenant moins de 10 % en poids de matière de départ oléfinique n'ayant pas réagi et moins de 10 % en poids d'oléfines polyéthylées ; que le mélange enrichi ainsi obtenu est hydroformylé avec formation d'alcanols ; et que les alcanols ainsi formés sont transformés en agent tensio-actif.

5. Un procédé selon la revendication 1 ou 2, caractérisé en ce que les oléfines éthylées sont transformées en agent tensio-actif sulfonate d'oléfine par réaction avec l'anhydride sulfurique.

6. Un procédé selon la revendication 1 ou 2, caractérisé en ce que la conversion oléfines éthylées en agent tensio-actif comprend une.étape d'alcoylation par des composés aromatiques pour faire réagir les oléfines éthylées avec un ou plusieurs composés aromatiques compris dans le groupe constitué par les phénols, le benzène, le toluène et les xylènes.

7. Un procédé selon la revendication 6, caractérisé en ce que la conversion des oléfines éthylées en agent tensio-actif comprend aussi une étape de sulfonation pour transformer les composés aromatiques alcoylés en sulfonates correspondants par réaction avec un agent de sulfonation.

8. Un procédé selon la revendication 6, caractérisé en ce que le corps en réaction aromatique est choisi dans le groupe constitué par les phénols et que la conversion des oléfines éthylées en agent tensio-actif comprend aussi une étape déthoxylation pour transformer les phénols alcoylés en éthoxylates correspondants par réaction avec l'oxyde d'éthylène,

9.  Un procédé selon la revendication 3 ou 4, caractérisé en ce que les alcanols sont transformés en agent tensio-actif sulfate d'alcanol par réaction avec un agent de sulfatation choisi dans le groupe constitué par l'anhydride sulfurique, l'acide chlorosulfonique, l'acide sulfamique et leurs mélanges.

10. Un procédé selon la revendication 3 ou 4, caractérisé en ce que les alcanols sont transformés en agent tensio-actif sulfonate d'éther d'alcoyl ou d'alcoyl éthoxy glycérol.

11. Un procédé selon la revendication 3 ou 4, caractérisé en ce que les alcanols sont transformés en agent tensio-actif alcoyl polysaccharide.

12. Un procédé selon la revendication 3 ou 4, caractérisé en ce que la conversion des alcanols en agents tensio-actifs comprend une étape pour faire réagir les alcanols avec un ou plusieurs réactifs choisis dans le groupe constitué par l'ammoniac et les alcoylamines de $C_1$ à $C_4$.